# EUROPÄISCHE PATENTSCHRIFT

(11) **EP 2 751 062 B1**
(45) Veröffentlichungstag und Bekanntmachung des Hinweises auf die Patenterteilung: **30.09.2015**
(21) Anmeldenummer: 12753690.2
(22) Anmeldetag: 23.08.2012
(51) Int. Cl.: C07C 67/055, C07C 69/01

(54) **VERFAHREN ZUR ACETOXYLIERUNG VON OLEFINEN IN DER GASPHASE**
METHOD FOR THE ACETOXYLATION OF OLEFINS IN THE GAS PHASE
PROCÉDÉ D'ACÉTOXYLATION D'OLÉFINES EN PHASE GAZEUSE

(30) Priorität: 30.08.2011 DE 102011081786
(43) Veröffentlichungstag der Anmeldung: 09.07.2014
(73) Patentinhaber: Wacker Chemie AG, 81737 München (DE)
(72) Erfinder: ROSCHER, Anja, 81379 München (DE); EBERLE, Hans-Jürgen, 81477 München (DE); HEIDENREICH, Roland, 81825 München (DE); JUNG, Herbert, 81675 München (DE)
(74) Vertreter: Killinger, Andreas
(86) Internationale Anmeldenummer: PCT/EP2012/066390
(87) Internationale Veröffentlichungsnummer: WO 2013/030073

(56) Entgegenhaltungen:
- EP-A2- 0 967 009
- EP-A2- 1 164 123
- WO-A2-2008/071610

## Beschreibung

Die vorliegende Erfindung betrifft ein Verfahren zur Acetoxylierung von Olefinen, bei dem man einen gasförmigen Reaktionsstrom, enthaltend ein Olefin, Essigsäure und molekularen Sauerstoff, über mindestens zwei nacheinander angeordnete Katalysatorschichten unterschiedlicher Reaktivität leitet. Weiterhin betrifft die Erfindung ein Katalysatorsystem zur Gasphasenoxidation von Olefinen zu acetoxylierten Produkten, insbesondere zur Herstellung von Vinylacetat, das aus mindestens zwei schichtweise angeordneten Katalysatorlagen mit an den Reaktionsverlauf angepasster Reaktivität besteht.

Von besonderem technischen Interesse sind insbesondere die Verfahren zur Acetoxylierung von Ethylen in der Gasphase. Aus der Literatur ist bekannt, dass Acetoxylierungen technisch durch katalytische Gasphasenoxidation von Olefinen, wie beispielsweise Ethen oder Propen, in Festbettreaktoren durchgeführt werden. Vorzugsweise werden diese Reaktionen in Rohrbündelreaktoren durchgeführt. Mit dieser Reaktion werden unter anderem ungesättigte Ester, wie beispielsweise Vinylacetat, hergestellt. Allgemein leitet man bei dieser Reaktion ein gasförmiges Gemisch bestehend aus molekularem Sauerstoff, einem Olefin und Essigsäure durch einen Reaktor. Üblicherweise verwendet man dabei einen sogenannten Rohrbündelreaktor, in dem eine Vielzahl von Reaktionsrohren parallel angeordnet sind und in denen sich jeweils eine einheitliche Katalysatorfüllung befindet. Die dabei entstehende überschüssige Reaktionswärme wird durch ein Wärmeträgermedium abgeführt. Ein Beispiel hierfür ist ein Siedewasserreaktor.

Als katalytisch aktive Komponenten der hier verwendeten Katalysatoren können u. a. Palladium und/oder dessen Verbindungen und Alkaliverbindungen eingesetzt werden, sowie zusätzlich Gold und/oder dessen Verbindungen (System Pd/Alkali/Au). Auch Systeme aus Cadmium und/oder dessen Verbindungen (System Pd/Alkali/Cd) oder Barium und/oder dessen Verbindungen (System Pd/Alkali/Ba) kommen neben Systemen enthaltend Palladium, Alkaliverbindungen und Mischungen aus Gold und/oder Cadmium und/oder Barium zum Einsatz. Alle diese Systeme liegen dabei üblicherweise auf einem geeigneten Trägermaterial vor. Bevorzugt werden industriell Palladium/Gold-Katalysatorsysteme eingesetzt.

Als Alkaliverbindungen werden üblicherweise Kaliumverbindungen, wie beispielsweise Kaliumacetat, eingesetzt. Während des Betriebs erfolgt üblicherweise eine Zudosierung der Alkaliverbindung um entsprechende Alkaliverluste innerhalb der Katalysatorschüttung auszugleichen.

Trotz verschiedener aus dem Stand der Technik bekannter Temperaturregelungen kann es in der Katalysatorschüttung zu einer lokalen Ausbildung einer stationären Temperaturspitze kommen, in der eine höhere Temperatur vorherrscht als im übrigen Teil der Katalysatorschüttung.

Diese Temperaturspitzen (sogenannte "hot spots") fördern eine Reihe von unerwünschten Effekten während des Reaktionsverlaufes. Zum einen limitieren sie eine weitere Erhöhung der Eduktkonzentration (Beladung), was gleichbedeutend mit einer Limitierung der Raum-Zeit-Leistung ist, zum anderen kann es zu einer Vermehrung einer Totaloxidation im Reaktionsgemisch kommen (Selektivitätsminderung für das Zielprodukt). Letzteres macht sich insbesondere in einem höheren spezifischen Rohstoffeinsatz bemerkbar und beeinflusst die Wirtschaftlichkeit eines Verfahrens signifikant negativ. Weiterhin fördern hot spots die vorzeitige Alterung des Katalysators.

Gerade bei frischen Katalysatorschüttungen sowie bei Reaktionsführungen mit hohen Sauerstoffbeladungen kann es in diesem sensiblen Bereich mit ungenügender Wärmeabführung zu einer irreversiblen Schädigung des Katalysators kommen.

Weiterhin kommt es im Verlauf des Reaktionsfortschritts entlang der Katalysatorschüttung zu einer Abnahme der Eduktkonzentration, verbunden mit einer gleichzeitigen Zunahme der Produktkonzentration. Letztere kann dann wiederum zu einer Produkthemmung und damit zu einer Verschlechterung der Selektivität und Umsatz führen.

Von wirtschaftlich großer Bedeutung sind Verfahren zur Acetoxylierung von Ethylen in der Gasphase, die zu hohen Ausbeuten an Vinylacetat führen.

WO 2008/071610 offenbart ein Verfahren und ein Katalysatorsystem mit einem auf einen SiO₂-Träger mit hoher Oberfläche aufgebrachten Katalysator, bestehend aus Palladium, Gold und Kaliumacetat, der mit einer Raum-Zeit-Ausbeute von mehr als 800 [g(VAM)/lKat*h] bei Ethen-Selektivitäten von mehr als 92 %, und bei niedriger Ethylacetatbildungsrate bezogen auf Vinylacetat gefahren werden kann.

Aus US 5 179056 ist ein Verfahren zur Herstellung von Vinylacetat durch Umsetzung von Ethylen und Essigsäure in Anwesenheit eines sauerstoffhaltigen Gases an einem hochreaktiven Palladium/Gold Schalenkatalysator bekannt.

US 6 399 813 offenbart einen hochaktiven Fließbett-Vinylacetat Katalysator auf einem Träger aus inerten microsphäroidalen Teilchen mit definierter Porenverteilung aus Silicium-, Zircon- oder Aluminiumoxid.

In Anbetracht der großen wirtschaftlichen Bedeutung von acetoxylierten Produkten und den auf dem Stand der Technik bekannten Hochleistungskatalysatoren besteht großer Optimierungsbedarf des Reaktionsverlaufes, hinsichtlich Umsatz, Selektivität und Lebensdauer des Katalysators.

Aus WO 2007/101749 sowie WO 2006/042659 sind beispielsweise Synthesereaktoren zur Herstellung von Vinylacetat-Monomer mit erhöhter Selektivität und Produktivität bekannt, in welchen gasförmiges Ethylen und Essigsäure sowie ein Sauerstoff enthaltendes Gas katalytisch reagieren, wobei der Synthesereaktor ein Wandreaktor ist und die katalytische Synthese in einer Vielzahl von Reaktionsräumen erfolgt und mindestens eine Wand der Reaktionsräume mit Katalysator beschichtet ist und mindestens eine Wand der Reaktionsräume indirekt gekühlt wird.

Ebenfalls sind aus dem Stand der Technik Reaktionen bekannt, die eine Hintereinanderschaltung von mindestens zwei Reaktoren beschreiben, die mit unterschiedlich reaktiven Katalysatoren befüllt sein können. Nachteilig an dieser Anordnung ist aber der große apparative Aufwand.

Die Aufgabe der Erfindung war es, ein neues Verfahren zur Acetoxilierung von Essigsäure mit einem Katalysatorsystem bereitzustellen, mit dem insbesondere hohe Raum-Zeit-Ausbeuten, hohe Selektivitäten bei geringer Nebenproduktbildung, sowie ein möglichst isothermes Temperaturprofil im Katalysatorbett erreicht werden, wodurch eine Verlängerung der Lebenszeit wie auch kurze Anfahrzeiten von frischem Katalysator erzielt werden können, und gleichzeitig die Verwendung von herkömmlichen Rohrbündelreaktoren beibehalten werden kann.

Überraschenderweise wurde gefunden, dass durch die Anwendung eines Katalysatorsystems, das aus mindestens zwei unterschiedlich reaktiven Katalysatorschichten besteht, welche schichtweise in Strömungsrichtung angeordnet werden, eine höhere Raum-Zeit-Ausbeute, bessere Selektivitäten bei geringer Nebenproduktbildung, verbunden mit einem nahezu isothermen Temperaturprofil im Katalysatorbett erreicht werden konnte.

Gegenstand der Erfindung ist ein Verfahren zur Acetoxylierung von Olefinen in einem gasförmigen Reaktionsstrom, enthaltend ein Olefin, Essigsäure und ein sauerstoffhaltiges Gas, dadurch gekennzeichnet, dass das Reaktionsgas über mindestens zwei nacheinander angeordnete Katalysatorlagen unterschiedlicher Reaktivität geleitet wird, wobei sich die Katalysatorlagen in einem oder mehreren nebeneinander angeordneten Reaktionsrohren befinden.

Als sauerstoffhaltiges Gas wird bevorzugt molekularer Sauerstoff verwendet.

Das erfindungsgemäße Verfahren kann zur Herstellung ungesättigter Ester aus Olefinen, organischen Säuren und Sauerstoff in der Gasphase verwendet werden. Insbesondere kann es für die Produktion von Vinylacetatmonomer verwendet werden. Dazu werden Ethen, Essigsäure und molekularer Sauerstoff, beziehungsweise Luft, in der Gasphase, gegebenenfalls unter Zusatz von Inertgasen, bei Temperaturen von 100 bis 250 °C und bei normalem bzw. erhöhtem Druck, zum Beispiel 1 bis 25 bar, in Gegenwart des erfindungsgemäßen mehrlagigen Katalysatorsystems zur Reaktion gebracht. Typischer Weise werden in technischen Rohrbündelreaktoren Raumgeschwindigkeiten in Bezug auf die Gasphase von 1000 bis 10000 Normliter Gasgemisch pro Liter Katalysator und pro Stunde realisiert.

Bei dem Verfahren zur Herstellung von Vinylacetatmonomer kann das Katalysatorsystem mit unterschiedlichen Aktivitäten der einzelnen Katalysatorlagen, gegenüber einer herkömmlichen einheitlichen Katalysatorlage von bestimmter Aktivität, schnell auf hohe Raum-Zeit-Leistung mit deutlich verbesserter Selektivität hochgefahren werden. Nach dem Stand der Technik erreichen einheitliche Katalysatoren mit einheitlicher Aktivität gewöhnlich erst nach Tagen oder Wochen ihr endgültiges Niveau.

Die erfindungsgemäße Anordnung des Katalysators kann auch zur Acetoxylierung von Olefinen, wie beispielsweise Propen, eingesetzt werden.

Es wurde überraschend gefunden, dass bereits die Aneinanderreihung von zwei Katalysatorlagen mit unterschiedlicher Aktivität bei der Herstellung insbesondere von Vinylacetat aus Ethen, Essigsäure und Sauerstoff in der Gasphase zu einer deutlichen Erhöhung der Raum-Zeit-Leistung und Selektivität führt. Erfindungsgemäß werden mindestens zwei Katalysatorlagen mit unterschiedlicher Aktivität aneinander gereiht. Eine Aneinanderreihung von mehr als zwei Lagen ist ebenfalls möglich und Teil dieser Erfindung.

Vorzugsweise weist die zur Gaseintrittseite hin gelegene Katalysatorlage eine niedrigere Aktivität auf und die sich daran anschließenden Katalysatorlagen besitzen in ihrer Reihenfolge bis hin zur Gasaustrittsseite eine steigende Aktivität.

Dadurch, dass auf der Gaseintrittsseite eine aktivitätsgedämpfte Katalysatorlage vorliegt, ist eine erhebliche Verkürzung der Zeitdauer erzielbar, die bei der ersten Inbetriebnahme des Katalysators benötigt wird, um die gewünschte maximale Raum-Zeit-Leistung unter stabilen Betriebsbedingungen zu erreichen.

Für das erfindungsgemäße Verfahren der Verwendung eines mehrlagigen Katalysators ist es unerheblich, ob der Katalysator in Form einer Katalysatorschüttung (beispielsweise einem Festbettkatalysator in einem Rohrbündelreaktor) vorliegt oder in Form von einzelnen Bereichen (beispielsweise relativ dünnen Katalysatorbeschichtungen in einer Mikrostruktur) aufgebracht ist.

Eine besonders bevorzugte Ausführungsform der Erfindung ist die Aneinanderreihung von drei Katalysatorlagen, wobei die zur Gaseintrittseite hin gelegene erste Katalysatorlage gegenüber der zur Gasaustrittseite hin gelegenen zweiten und dritten Katalysatorlagen die niedrigste Aktivität, und die dritte Katalysatorlage die höchste Aktivtät aufweist. Dieses Katalysatorsystem mit einem Aktivitätsgradienten von der Gaseintrittseite hin zur Gasaustrittsseite führt hinsichtlich Raum-Zeit-Leistung und Selektivitäten zu weiteren erheblichen Verbesserungen.

Zur Steuerung der Aktivität der einzelnen Katalysatorschichten können eine oder mehrere der folgenden Maßnahmen verwendet werden:
a.) Variation des Gehaltes an aktivitätssteuerenden Promotoren im Katalysator (Kaliumacetat, Lithiumacetat, Natriumacetat, Cäsiumacetat, Rubidiumacetat oder Mischungen aus diesen)
b.) Einsatz unterschiedlicher Metallgehalte an Gold, Palladium, Cadmium bezogen auf den Katalysatorträger
c.) Variation des Mischungsverhältnisses der Edelmetalle, wie beispielsweise Pd:Au
d.) Verwendung unterschiedlicher BET-Oberflächen der verwendeten Katalysatorträger
e.) Einsatz verschiedener Schüttdichten der Katalysatorträger
f.) Einsatz verschiedener Katalysatorträgergeometrien
g.) Verwendung unterschiedlicher Metalldispersionen
h.) Verdünnung eines zu aktiven/"super-aktiven" Vinylacetatmonomer-Katalysators mittels Inertmaterial
i.) Verwendung unterschiedlicher Acidität und/oder Hydrophilie des Katalysatorträgers
j.) Variation der Dicke der aktiven Edelmetallschicht auf dem Katalysatorträger.

Als Trägerformen der eingesetzten Katalysatoren können alle dem Fachmann bekannten Geometrien eingesetzt werden, wie beispielsweise Kugeln, Zylinder, Ringe, Formkörper mit einem oder mehreren Durchtrittskanälen, Kronenringe, Wagenräder, Monolithe, Trilobes oder Tetralobes.

Bestimmte Katalysatorformkörper, wie beispielsweise Ringe, Kronenringe, Ringe mit mehreren Durchtrittskanälen, ermöglichen niedrige Druckverluste sowie niedrige Schüttdichten.

In einer Ausführung der Erfindung werden in dem mehrschichtigen Katalysatorsystem verschiedene Geometrien von Katalysatorformkörpern eingesetzt. Besonders bevorzugt ist eine Ausführung bei der eine oder mehrere Katalysatorschichten dabei aus Ringen mit einer Wandstärke kleiner 2 mm, besonders bevorzugt kleiner 1 mm besteht.

Mit Hilfe von mehrlagigen Katalysatorsystemen sowie unterschiedlicher Katalysatorformkörpergeometrien kann das Wärmemanagement, abhängig von der Rohrgeometrie des Reaktors hervorragend angepasst werden.

Insbesondere werden VAM-Katalysatoren auf SiO₂ -Trägern oder Mischoxid-Trägern verwendet, wobei die eingesetzten Metalloxide natürlichen Ursprungs, z.B. Bentonite, sein können öder pyrogen hergestellt wurden. Besonders bevorzugt sind SiO₂-Träger, insbesondere SiO₂-Träger auf Basis von pyrogener Kieselsäure. Ferner ist es möglich, dass diese Träger durch gezielte Dotierung modifiziert oder durch Waschen vorbehandelt werden.

Als Katalysatorträger können hoch-oberflächige, hoch-reine Siliziumdioxidträger wie in WO 2008/071610 beschrieben verwendet werden, die BET-Oberflächen zwischen 30 m²/g und 500 m²/g aufweisen. Ebenso können gezielt metall-dotierte SiO₂-Materialien verwendet werden.

Für die Synthese von Vinylacetatmonomer ist es zweckmäßig, den Katalysator mit 0,1 bis 5,0 Gew.-% Palladium und 0,2 bis 3,5 Gew.-% Gold oder 0,1 bis 3,5 Gew.-% Cadmium oder 0,1 bis 3,5 Gew.-% Barium und 0,5 bis 15 Gew.-% Alkali, jeweils bezogen auf das Gewicht des eingesetzten Trägers, zu belegen. Die Beladungen können dabei je nach eingesetztem Katalysatortyp (Pd/Au-Typ, Pd/Cd-Typ oder Pd/Ba-Typ), variieren.

Der Palladium-Gehalt der Pd/Alkali/Au-Katalysatoren beträgt 0,2 bis 5,0 Gew.-%, bevorzugt 0,3 bis 3,0 Gew.-%.,
Der Gold-Gehalt der Pd/Alkali/Au-Katalysatoren beträgt 0,2 bis 5,0 Gew.-%, bevorzugt 0,3 bis 3,0 Gew.-%.
Der Alkali-Gehalt der Pd/Alkali/Au-Katalysatoren beträgt 0,5 bis 15 Gew.-%, vorzugsweise 1,0 bis 10 Gew.-%.
Der Palladium-Gehalt der Pd/Alkali/Cd beziehungsweise Pd/Alkali/Ba-Katalysatoren beträgt 0,1 bis 5 Gew.-%, bevorzugt 0,2 bis 4,0 Ges.-%.
Der Cadmium-Gehalt der Pd/Alkali/Cd-Katalysatoren beträgt 0,1 bis 3,5 Gew.-%, vorzugsweise 0,2 bis 3,0 Gew.-%.
Der Barium-Gehalt der Pd/Alkali/Ba-Katalysatoren beträgt 0,1 bis 3,5 Gew.-%, vorzugsweise 0,2 bis 3,0 Gew.-%. Der Ba-Gehalt liegt dabei vorzugsweise im gleichen Bereich wie der Cd-Gehalt bei Cd-Typen.
Der Alkali-Gehalt der Pd-Alkali/Cd- bzw. Pd/Alkali/Ba-Katalysatoren beträgt 0,3 bis 15 Gew.-%, vorzugsweise 0,5 bis 10 Gew.-%.

Anhand der folgenden Beispiele soll die Erfindung näher erläutert werden.

### Allgemeine Testbedingungen:

Aktivität und Selektivität der Katalysatoren aus den folgenden Beispielen und Vergleichsbeispielen werden über die Dauer von bis zu 200 Stunden gemessen. Die Katalysatoren werden in einem Öl temperierten Strömungsrohr (Reaktorlänge 1200 mm, Innendurchmesser 19 mm) bei einem absoluten Druck von 9,8 bar und einer Raumgeschwindigkeit (GHSV) von 4000 - 5000 Nm³ / (m³*h) mit folgender Gaszusammensetzung geprüft: 60 Vol.-% Ethen, 19,5 Vol.-% Argon, 13 Vol.-% Essigsäure und 7,5 Vol.-% Sauerstoff. Die Katalysatorsysteme werden im Temperaturbereich von 130 bis 180 °C (Gaseintrittstemperatur vor Katalysatorbett) getestet. Um den Reaktionsverlauf zu charakterisieren wird das Temperaturprofil mit Hilfe eines Multipoint-Thermoelements im Katalysatorbett erfasst. Die Reaktionsprodukte und nicht umgesetzte Edukte werden im Ausgang des Reaktors mittels online Gaschromatographie analysiert. Als Maß für die Katalysatoraktivität wird die Raum-Zeit-Ausbeute des Katalysatorsystems in Gramm Vinylacetatmonomer pro Stunde und Liter Katalysator (g(VAM)/lKat.*h) bestimmt. Die Selektivität wird durch das Verhältnis von gebildetem Vinylacetat zu umgesetztem Ethen bestimmt.

Zusätzlich zur Bestimmung der Reaktionsprodukte in der Gasphase werden die flüssigen Reaktionsprodukte in einem auf 10 bis 15 °C temperierten Behälter kondensiert und das erhaltene Kondensat mittels Gaschromatographie analysiert.

### Herstellung der Katalysatoren:

Die Herstellung und Charakterisierung der einzelnen Katalysatoren für die Beispiele sowie Vergleichsbeispiele ist in WO 2008/071610 ausführlich beschrieben. Die BET-Oberfläche der Katalysatorträger wird gemäß DIN 66131 mit Stickstoff bestimmt.

Die Angaben der Füllhöhen der einzelnen Schichten beziehen sich auf die Gesamtlänge (100 %) des beschriebenen Testreaktors. Je nach Reaktionsrohrdimensionierung können sich andere optimale Füllhöhen ergeben.

Die Angaben der Katalysatorlagen beziehen sich auf die Lage hinsichtlich der Strömungsrichtung des Gases, womit als erste Lage die Katalysatorschicht am Gaseintritt des Reaktors gemeint ist.

Für das erfindungsgemäße Mehrlagenkatalysatorsystem ist es unerheblich, ob bei einem Reaktor (beispielsweise einem stehenden Rohrbündelreaktor) die Reaktionsstoffe von unten nach oben oder von oben nach unten strömen.

### Beispiel 1: (Katalysator Pd/Au-Typ mit unterschiedlichem Promotorengehalt)

Hierzu wurde ein Katalysator gemäß WO 2008/071610 hergestellt mit der Ausnahme, dass der Katalysator der 1. Lage 6,5 Gew.-% Kalium und der Katalysator in der 2. Lage 3,0 Gew.-% Kalium enthält.

| Bezeichnung | 1. Lage | 2. Lage | 3. Lage |
|---|---|---|---|
| relative Länge [%] | 50 | 50 | - |
| SiO₂-Träger BET (m2/g) | 200 | 200 | - |
| Pd (Gew.-%) | 2,0 | 2,0 | - |
| Au (Gew.-%) | 2,0 | 2,0 | - |
| K (Gew.-%) | 6,5 | 3,0 | - |

Mit diesem erfindungsgemäßen Katalysatorsystem lassen sich beim Test im Reaktor unter den beschriebenen Bedingungen max. Raum-Zeit-Leistungen von 980 g(VAM)/lKat.*h bei max. Ethen-Selektivitäten von 93,2 % erreichen.

### Beispiel 2: (Katalysator Pd/Au Typ mit unterschiedlicher BET Trägeroberfläche und Promotorengehalt)

Es wurden zwei Katalysatoren gemäß WO 2008/071610 synthetisiert, die sich darin unterscheiden, dass der Katalysator in der 1. Lage auf SiO₂-Trägern mit einer BET Oberfläche von 150 m²/g und einem Kaliumgehalt von 6,5 Gew.-% und der Katalysator in der 2. Lage auf SiO₂-Trägern mit einer BET Oberfläche von 220 m²/g und einem Kaliumgehalt von 3,0 Gew.-% hergestellt wurden.

| Bezeichnung | 1. Lage | 2. Lage | 3. Lage |
|---|---|---|---|
| relative Länge [%] | 50 | 50 | - |
| SiO₂-Träger BET (m2/g) | 150 | 220 | - |
| Pd (Gew.-%) | 2,0 | 2,0 | - |
| Au (Gew.-%) | 2,0 | 2,0 | - |
| K (Gew. -%) | 6,5 | 3,0 | - |

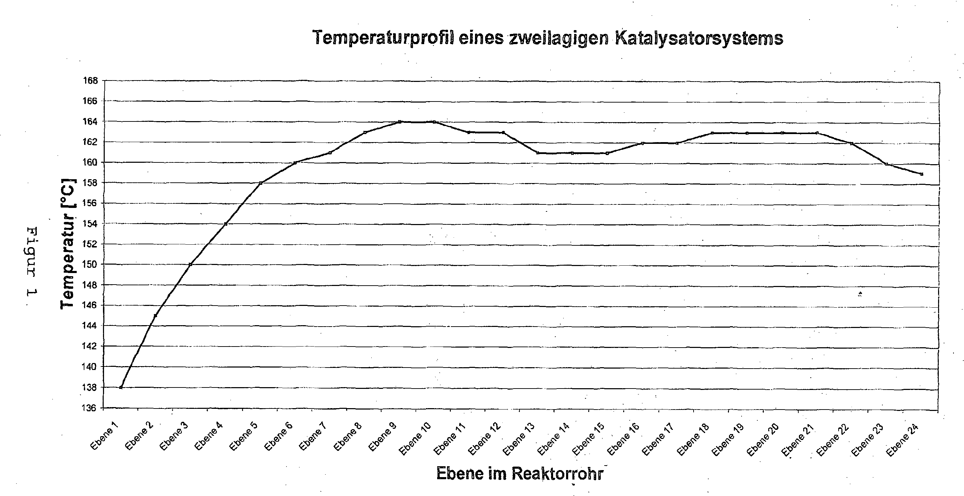

Mit diesem erfindungsgemäßen Katalysatorsystem lassen sich beim Test im Reaktor unter den beschriebenen Bedingungen max. Raum-Zeit-Leistungen von 1050 g(VAM)/lKat.*h bei max. Ethen-Selektivitäten von 93,5 % erreichen. Figur 1 zeigt das Temperaturprofil dieses Zweischicht-Katalysatorsystems mit einem weitgehend isothermen Temperaturverlauf unter optimierter Katalysator Ausnutzung im hinteren Bereich des Reaktors. Figur 1 zeigt das Temperaturprofil bei 7,5 Vol.-% O₂, 4500 Nm3 / (m^{3*}h) und einer Gaseintrittstemperatur von 138 °C.

### Beispiel 3: (Katalysator Pd/Au Typ mit unterschiedlichem EM-Gehalt)

Es wurden zwei Katalysatoren gemäß WO 2008/071610 synthetisiert, die sich darin unterscheiden, dass der Katalysator in der 1. Lage auf SiO₂-Trägern mit einer BET Oberfläche von 180 m²/g und einem Pd- und Au-Gehalt von jeweils 2,0 Gew.-% und der Katalysator in der 2. Lage auf SiO₂-Trägern mit einer BET Oberfläche von 220 m²/g und einem Pd- und Au-Gehalt von jeweils 2,3 Gew.-% hergestellt wurden.

| Bezeichnung | 1. Lage | 2. Lage | 3. Lage |
|---|---|---|---|
| relative Länge [%] | 60 | 40 | - |
| SiO₂-Träger BET (m²/^{g}) | 180 | 220 | - |
| Pd (Gew.-%) | 2,0 | 2,3 | - |
| Au (Gew.-%) | 2,0 | 2,3 | - |
| K (Gew. -%) | 6,5 | 3,0 | - |

Mit diesem erfindungsgemäßen Katalysatorsystem lassen sich beim Test im Reaktor unter den beschriebenen Bedingungen max. Raum-Zeit-Leistungen von 1200 g(VAM)/lKat.*h bei Ethen-Selektivitäten von 94,0 % erreichen.

### Beispiel 4: (Drei Katalysatorlagen mit unterschiedlichen Trägergeometrien)

Es wurden drei Katalysatoren gemäß WO 2008/071610 synthetisiert, die sich durch unterschiedliche BET Oberflächen der SiO₂-Träger EM- und Kalium-Gehalte unterscheiden sowie durch eine verringerte Wandstärke in der 3. Lage.

| Bezeichnung | 1. Lage | 2. Lage | 3. Lage |
|---|---|---|---|
| relative Länge [%] | 50 | 30 | 20 |
| SiO₂-Träger BET (m²/g) | 200 | 200 | 220 |
| Trägergeomtrie Wandstärke [mm] | Ringe | Ringe | Ringe |
| | 1,8 | 1,8 | 1,3 |
| Pd (Gew.-%) | 2,0 | 2,3 | 2,6 |
| Au (Gew.-%) | 2,0 | 2,3 | 2,6 |
| Cd (Gew.-%) | 0 | 0 | 0 |
| K (Gew.-%) | 6,5 | 3,5 | 2,0 |

Mit diesem erfindungsgemäßen Katalysatorsystem lassen sich beim Test im Reaktor unter den beschriebenen Bedingungen max. Raum-Zeit-Leistungen von 1250 g(VAM)/lKat.*h bei max. Ethen-Selektivitäten von 94,5 % erreichen.

### Beispiel 5: (Zwei Katalysatorlagen mit geringer Wandstärke)

Es wurden zwei Katalysatoren gemäß WO 2008/071610 synthetisiert, die sich durch unterschiedliche Wandstärken der SiO₂-Träger und Kalium-Gehalte unterscheiden.

| Bezeichnung | 1. Lage | 2. Lage | 3. Lage |
|---|---|---|---|
| relative Länge [%] | 60 | 40 | - |
| SiO₂-Träger BET (m²/g) | 200 | 200 | - |
| Trägergeomtrie Wandstärke [mm] | Ringe | Ringe | - |
| | 0,6 | 1,3 | |
| Pd (Gew.-%) | 2,0 | 2,3 | - |
| Au (Gew.-%) | 2,0 | 2,3 | - |
| Cd (Gew.-%) | 0 | 0 | - |
| K (Gew. -%) | 5,5 | 2,5 | - |

Mit diesem erfindungsgemäßen Katalysatorsystem lassen sich beim Test im Reaktor unter den beschriebenen Bedingungen max. Raum-Zeit-Leistungen von 1100 g(VAM)/lKat.*h bei max. Ethen-Selektivitäten von 94,0 % erreichen.

### Vergleichsbeispiel 1: (Katalysator Pd/Au-Typ)

Es wurde ein Katalysator gemäß der Beschreibung in WO 2008/071610 hergestellt, der folgende Zusammensetzung aufweist:

| Bezeichnung | 1. Lage | 2. Lage | 3. Lage |
|---|---|---|---|
| relative Länge [%] | 100 | - | - |
| SiO₂-Träger BET (m²/g) | 200 | - | - |
| Pd (Gew.-%) | 2,0 | - | - |
| Au (Gew.-%) | 2,0 | - | - |
| K (Gew.-%) | 6,5 | - | - |

Mit diesem nicht-erfindungsgemäßen (einlagigen) Katalysator lassen sich beim Test im Reaktor unter den beschriebenen Testbedingungen max. Raum-Zeit-Leistungen von 850 g(VAM)/lKat.*h bei max. Ethen-Selektivitäten von 92,0 % erreichen.

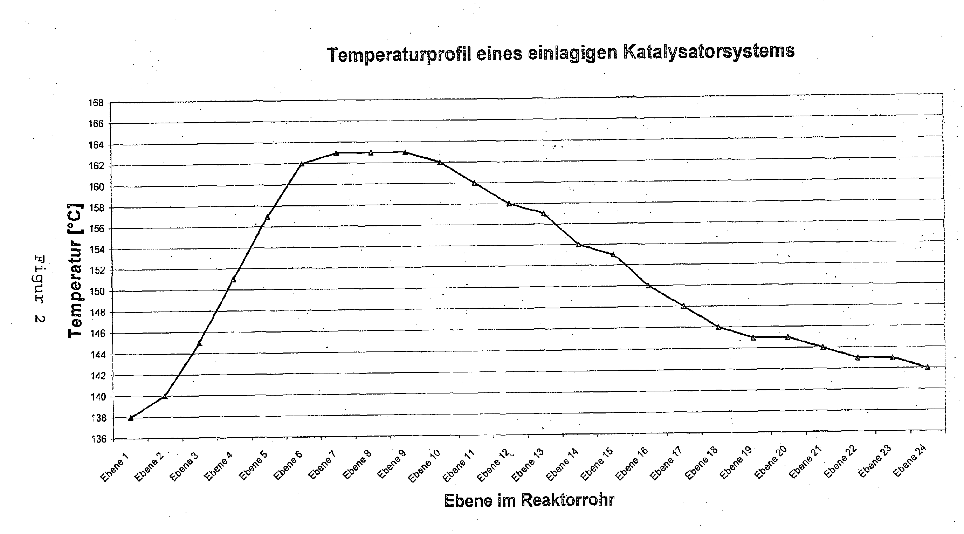

In Figur 2 ist das Temperaturprofil eines nicht-erfindungsgemäßen Katalysators (einlagig) gezeigt, mit deutlicher Ausbildung eines Hot-Spots im vorderen Reaktorbereich. Das Temperaturprofil zeigt das einlagige Katalysatorsystem bei 7,5 Vol.-% O₂, 4500 Nm₃ / (m³*h) und einer Gaseintrittstemperatur von 138 °C.

Die Ergebnisse der einzelnen Beispiele (Bsp.) und Vergleichsbeispiele (Vgl. Bsp.) sind in Tabelle 1 zusammengestellt.

**Tabelle 1:**

| Bezeichnung | Vergl. Bsp. 1 | Bsp. 1 | Bsp. 2 | Bsp. 3 | Bsp. 4 | Bsp. 5 |
|---|---|---|---|---|---|---|
| Katalysatorfüllhöhe [%] | | | | | | |
| 1. Lage | 100 | 50 | 50 | 60 | 50 | 60 |
| 2. Lage | - | 50 | 50 | 40 | 30 | 40 |
| 3. Lage | - | - | - | - | 20 | - |
| RZA g(VAM)/lKat. *h | 850 | 980 | 1050 | 1200 | 1250 | 1100 |
| Selektivität Ethen [%] | 92,0 | 93,2 | 93,5 | 94,0 | 94,5 | 94,0 |
| Hot Spot [°C] | | | | | | |
| 1. Lage | 163 | 164 | 164 | 170 | 172 | 165 |
| 2. Lage | - | 163 | 163 | 163 | 166 | 162 |
| 3. Lage | - | - | - | - | 164 | - |

## Patentansprüche

1. Verfahren zur Acetoxylierung von Olefinen in einem gasförmigen Reaktionsstrom, enthaltend ein Olefin, Essigsäure und ein sauerstoffhaltiges Gas, **dadurch gekennzeichnet, dass** das Reaktionsgas über mindestens zwei nacheinander angeordnete Katalysatorlagen unterschiedlicher Reaktivität geleitet wird, wobei sich die Katalysatorlagen in einem oder mehreren nebeneinander angeordneten Reaktionsrohren befinden.

2. Verfahren nach Anspruch 1, **dadurch gekennzeichnet, dass** als sauerstoffhaltiges Gas molekularer Sauerstoff verwendet wird.

3. Verfahren nach Anspruch 1 bis 2, **dadurch gekennzeichnet, dass** Ethen und Essigsäure zu Vinylacetatmonomer umgesetzt werden.

4. Verfahren nach Anspruch 1 bis 3, **dadurch gekennzeichnet, dass** die zur Gaseintrittseite hin gelegene Katalysatorlage eine niedrigere Aktivität aufweist und die sich daran anschließenden Katalysatorlagen in ihrer Reihenfolge bis hin zur Gasaustrittsseite eine steigende Aktivität aufweisen.

5. Verfahren nach Anspruch 1 bis 4, **dadurch gekennzeichnet, dass** die Steuerung der Aktivität der einzelnen Katalysatorschichten durch eine oder mehrere der folgenden Maßnahmen erfolgt:
a.) Variation des Gehaltes an aktivitätssteuerenden Promotoren im Katalysator (Kaliumacetat, Lithiumacetat, Natriumacetat, Cäsiumacetat, Rubidiumacetat oder Mischungen aus diesen)
b.) Einsatz unterschiedlicher Metallgehalte an Gold, Palladium, Cadmium bezogen auf den Katalysatorträger
c.) Variation des Mischungsverhältnisses der Edelmetalle, wie beispielsweise Pd:Au
d.) Verwendung unterschiedlicher BET-Oberflächen der verwendeten Katalysatorträger
e.) Einsatz verschiedener Schüttdichten der Katalysatorträger
f.) Einsatz verschiedener Katalysatorträgergeometrien
g.) Verwendung unterschiedlicher Metalldispersionen
h.) Verdünnung eines zu aktiven/"super-aktiven" Vinylacetatmomomer-Katalysators mittels Inertmaterial
i.) Verwendung unterschiedlicher Acidität und/oder Hydrophilie des Katalysatorträgers
j.) Variation der Dicke der aktiven Edelmetallschicht auf dem Katalysatorträger.

6. Verfahren nach Anspruch 1 bis 5, **dadurch gekennzeichnet, dass** die Form des Katalysatorträgers in einer oder mehreren Katalysatorschichten in Form von Kugeln, Zylindern, Ringen, Formkörpern mit einem oder mehreren Durchtrittskanälen, Kronenringen, Wagenrädern, Monolithen, Trilobes oder Tetralobes vorliegt.

7. Verfahren nach Anspruch 6, **dadurch gekennzeichnet, dass** Ringe mit einer Wandstärke kleiner 2 mm verwendet werden.

8. Verfahren nach Anspruch 1 bis 7, **dadurch gekennzeichnet, dass** die verwendeten Katalysatoren auf SiO₂-Trägern oder Mischoxid-Trägern vorliegen, wobei die eingesetzten Metalloxide natürlichen Ursprungs sein können oder pyrogen hergestellt wurden.

## Claims

1. Process for the acetoxylation of olefins in a gaseous reaction stream containing an olefin, acetic acid and an oxygen-containing gas, wherein the reaction gas is passed over at least two catalyst zones of differing reactivity arranged in series, where the catalyst zones are located in one or more reaction tubes arranged in parallel.

2. Process according to Claim 1, wherein molecular oxygen is used as oxygen-containing gas.

3. Process according to Claim 1 or 2, wherein ethene and acetic acid are reacted to form vinyl acetate monomer.

4. Process according to any of Claims 1 to 3, wherein the catalyst zone nearest the gas entry end has a lower activity and the adjoining catalyst zones have an increasing activity in order through to the gas exit end.

5. Process according to any of Claims 1 to 4, wherein the activity of the individual catalyst zones is controlled by means of one or more of the following measures:
a.) variation of the content of activity-controlling promoters in the catalyst (potassium acetate, lithium acetate, sodium acetate, cesium acetate, rubidium acetate or mixtures of these)
b.) use of different metal contents of gold, palladium, cadmium based on the catalyst support
c.) variation of the mixing ratio of the noble metals, for example Pd:Au
d.) use of different BET surface areas of the catalyst supports used
e.) use of various bulk densities of the catalyst supports
f.) use of various catalyst support geometries
g.) use of different metal dispersions
h.) dilution of a vinyl acetate monomer catalyst which is too active/"super-active" by means of inert material
i.) use of a differing acidity and/or hydrophilicity of the catalyst support
j.) variation of the thickness of the active noble metal layer on the catalyst support.

6. Process according to any of Claims 1 to 5, wherein the shape of the catalyst support in one or more catalyst zones is in the form of spheres, cylinders, rings, shaped bodies having one or more through-channels, crown rings, wagon wheels, monoliths, trilobes or tetralobes.

7. Process according to Claim 6, wherein rings having a wall thickness of less than 2 mm are used.

8. Process according to any of Claims 1 to 7, wherein the catalysts used are present on SiO₂ supports or mixed oxide supports, with the metal oxides used being able to be of natural origin or be pyrogenically produced.

## Revendications

1. Procédé pour l'acétoxylation d'oléfines dans un flux réactionnel gazeux, contenant une oléfine, de l'acide acétique et un gaz contenant de l'oxygène, **caractérisé en ce que** le gaz de réaction est guidé sur au moins deux couches catalytiques disposées l'une derrière l'autre de réactivité différente, les couches catalytiques se trouvant dans un ou plusieurs tubes de réaction disposés les uns à côté des autres.

2. Procédé selon la revendication 1, **caractérisé en ce qu'**on utilise, comme gaz contenant de l'oxygène, de l'oxygène moléculaire.

3. Procédé selon la revendication 1 à 2, **caractérisé en ce qu'**on transforme de l'éthylène et de l'acide acétique en monomère d'acétate de vinyle.

4. Procédé selon la revendication 1 à 3, **caractérisé en ce que** la couche catalytique située vers le côté de l'entrée des gaz présente une activité inférieure et les couches catalytiques consécutives présentent, dans leur ordre jusqu'au côté de sortie des gaz, une activité croissante.

5. Procédé selon la revendication 1 à 4, **caractérisé en ce que** la régulation de l'activité des différentes couches catalytiques a lieu par une ou plusieurs des mesures suivantes :
a.) variation de la teneur en promoteurs régulant l'activité dans le catalyseur (acétate de potassium, acétate de lithium, acétate de sodium, acétate de césium, acétate de rubidium ou mélanges de ceux-ci)
b.) utilisation de teneurs métalliques différentes en or, en palladium, en cadmium par rapport au support du catalyseur
c.) variation des rapports de mélange des métaux nobles, tels que par exemple Pd:Au
d.) utilisation de surfaces BET différentes des supports de catalyseur utilisés
e.) utilisation de densités apparentes différentes des supports de catalyseur
f.) utilisation de géométries différentes du support des catalyseurs
g.) utilisation de dispersions métalliques différentes
h.) dilution d'un catalyseur de monomère d'acétate de vinyle trop actif/"superactif" par un matériau inerte
i.) utilisation d'une acidité et/ou d'une hydrophilie différente(s) du support de catalyseur
j.) variation de l'épaisseur de la couche de métal noble actif sur le support de catalyseur.

6. Procédé selon la revendication 1 à 5, **caractérisé en ce que** la forme du support de catalyseur dans l'une ou plusieurs couches catalytiques se trouve sous forme de billes, de cylindres, d'anneaux, de corps façonnés présentant un ou plusieurs canaux de passage, de bagues formant couronne, de roues, de monolithes, de trilobes ou de tétralobes.

7. Procédé selon la revendication 6, **caractérisé en ce qu'**on utilise des anneaux présentant une épaisseur de paroi inférieure à 2 mm.

8. Procédé selon la revendication 1 à 7, **caractérisé en ce que** les catalyseurs utilisés se trouvent sur des supports de SiO₂ ou d'oxydes mixtes, les oxydes métalliques utilisés pouvant être d'origine naturelle ou ayant été fabriqués par voie pyrogène.
